# EUROPEAN PATENT SPECIFICATION

(11) **EP 1 290 194 B1**
(45) Date of publication and mention of the grant of the patent: **26.05.2010**
(21) Application number: 00941529.0
(22) Date of filing: 14.06.2000
(51) Int. Cl.: C12N 15/57, C12N 15/75, C12N 15/01, C12N 9/28, C12N 9/56, C12N 1/21, C07K 14/32, C12P 21/02, C12R 1/07, C12R 1/19

(54) **PRODUCTION OF SECRETED POLYPEPTIDES**
HERSTELLUNG VON SEKRETIERTEN POLYPEPTIDEN
PRODUCTION DE POLYPEPTIDES SECRETES

(43) Date of publication of application: 12.03.2003
(73) Proprietor: GENENCOR INTERNATIONAL, INC., Palo Alto, California 94304-1013 (US)
(72) Inventor: DIAZ-TORRES, Maria, Los Gatos, CA 95030 (US)
(74) Representative: Kiddle, Simon John
(86) International application number: PCT/US2000/016764
(87) International publication number: WO 2001/096577

(56) References cited:
- EP-A2- 1 141 310
- WO-A-00/39323
- WO-A-98/11203
- WO-A-99/27107
- MATHIOPOULOS C ET AL: "A BACILLUS-SUBTILIS DIPEPTIDE TRANSPORT SYSTEM EXPRESSED EARLY DURING SPORULATION" MOLECULAR MICROBIOLOGY, vol. 5, no. 8, 1991, pages 1903-1914, XP000982568 ISSN: 0950-382X cited in the application
- SLACK F J ET AL: "TRANSCRIPTIONAL REGULATION OF A BACILLUS-SUBTILIS DIPEPTIDE TRANSPORT OPERON" MOLECULAR MICROBIOLOGY, vol. 5, no. 8, 1991, pages 1915-1926, XP000982589 ISSN: 0950-382X cited in the application
- PODBIELSKI A ET AL: "MOLECULAR CHARACTERIZATION OF GROUP A STREPTOCOCCAL (GAS) OLIGOPEPTIDE PERMEASE (OPP) AND ITS EFFECT ON CYSTEINE PROTEASE PRODUCTION" MOLECULAR MICROBIOLOGY,GB,BLACKWELL SCIENTIFIC, OXFORD, vol. 21, no. 5, 1996, pages 1087-1099, XP000906862 ISSN: 0950-382X cited in the application
- PODBLELSKI A ET AL: "THE GROUP A STREPTOCOCCAL DIPEPTIDE PERMEASE (DPP) IS INVOLVED IN THE UPTAKE OF ESSENTIAL AMINO ACIDS AND AFFECTS THE EXPRESSION OF CYSTEINE PROTEASE" MOLECULAR MICROBIOLOGY,GB,BLACKWELL SCIENTIFIC, OXFORD, vol. 28, no. 6, 1998, pages 1323-1334, XP000906863 ISSN: 0950-382X

## Description

### Field of the Invention

The present invention relates to the increased production of proteins, preferably, heterologous secreted proteins and cells having interrupted peptide transport activity.

### Background

Secretion of heterologous polypeptides is a widely used technique in industry. A cell can be transformed with a nucleic acid encoding a heterologous polypeptide of interest to be secreted and thereby produce large quantities of desired polypeptides. This technique can be used to produce a vast amount of polypeptide over what would be produced naturally. Polypeptides of interest have a number of industrial applications, including therapeutic and agricultural uses, as well as use in foods, cosmetics, cleaning compositions, animal feed, etc.

Thus, increasing secretion of polypeptides is of interest. Secretion of polypeptides into periplasmic space or into their culture media is subject to a variety of parameters. Typically, vectors for secretion of a polypeptide of interest are engineered to position DNA encoding a secretory signal sequence 5' to the DNA encoding the DNA of interest.

Attempts to increase secretion have often fallen into one of the following three areas: trying several different signal sequences, mutating the signal sequence, and altering the secretory pathway within the host. While some success has been found with the above methods, generally, they are time consuming and novel methods are desirable. Therefore, a problem to be solved is how to produce and/or secrete more proteins without solely relying on altering the signal sequence.

The instant invention provides a novel approach to improving secretion of polypeptides in a cell. Also provided herein are novel compositions useful in the methods of polypeptide secretion provided herein, and methods of making such compositions.

WO00139323 describes methods for the production of proteins in gram positive microorganisms containing a mutation in at least one of the genes of the opp operon cluster. This document is available for the assessment of novelty only.

### Summary of the Invention

In one aspect of the invention, a method of increasing secretion of a polypeptide in a cell is provided, wherein, said cell selected would express at least one peptide transport protein and the method comprises inactivating said at least one peptide transport protein in said cell and culturing said cell under conditions suitable for expression and secretion of said polypeptide, according to the claims.

The methods provided herein are applicable for production or secretion of polypeptides in a variety of cell types, wherein said cell is a member of the family *Bacillus*. In a preferred embodiment, said cell is a member of the family *Bacillus* wherein said member of the family *Bacillus* is selected from the group consisting of *B. licheniformis, B. lentus, S. brevis, B. stearothermophilus, B. alkalophilus, B. amyloliquefaciens, B. coagulans, B. circulans, B. lautus, B. thuringiensis, B. methanolicus* and *B. anthracis.*

The polypeptide which is secreted or produced by the methods provided herein can be any polypeptide. In a preferred embodiment, it is a heterologous polypeptide. In one aspect, the polypeptides is selected from the group consisting of hormone, enzyme, growth factor and cytokine. In one embodiment, the polypeptide is an enzyme, preferably selected from the group consisting of proteases, carbohydrases, reductases, lipases, isomerases, transferases, kinases, phophatases, cellulase, endo-glucosidase H, oxidase, alpha-amylase, glucoamylase, lignocellulose hemicellulase, pectinase and ligninase. In one aspect, said polypeptide is a bacillus protease, preferably subtilisin. In another aspect, said polypeptide is an amylase, preferably, bacillus amylase.

The peptide transport protein can be inactivated in a variety of ways. The peptide transport protein is a gene product of a dciA operon. It may be the gene product of the dciAE gene. The protein can be inactivated at the protein or nucleic acid level. In one aspect, the protein is inactivated because the gene encoding said protein has been mutated. In another embodiment, the operon comprising said gene has been mutated. The mutation can be caused in a variety of ways including one or more frameshifts, insertions, substitutions and deletions, or combinations thereof. The deletion can be of a single nucleotide or more, including deletion of the entire gene.

In another aspect of the invention, a method for producing a polypeptide in a Bacillus cell is provided which comprises the steps of obtaining a Bacillus cell comprising nucleic acid encoding a polypeptide to be produced, said cell further comprising a peptide transport operon wherein at least one gene product of said operon is inactive in said cell, and culturing said cell under conditions suitable for expression such that said polypeptide is produced, according to the claims. The peptide transport operon is a dciA operon. The gene product of said operon can be inactivated at the nucleic acid or protein level. Preferably, the inactivated gene product is encoded by dciAA or dciAE. In another aspect of the invention provided herein is a method of providing a Bacillus cell having increased polypeptide secretion, comprising inactivating a polypeptide transport operon in said cell according to the claims. Said operon is a dciA operon. In one embodiment, said operon has been mutated to inactivate a gene product of a dciAA and/or dciAE gene.

### Brief Description of the Drawings

Figures 1A and 1B show an embodiment of a nucleic acid (SEQ ID NO:1) encoding a dciA operon, wherein the nucleotides encoding dciAA and dciAE are shown, respectively, underlined and in bold.
Figure 2 shows an embodiment of an amino acid sequence for dciAA, encoded by nucleotides 210-1034 of the nucleic acid sequence shown in Figures 1A and 1B, wherein the amino acids shown in underlined are omitted in an embodiment of inactive dciAA provided herein.
Figure 3 shows an embodiment of an amino acid sequence for dciAB, encoded by nucleotides 1051-19T7 of the nucleic acid sequence shown in Figures 1A and 1B.
Figure 4 shows an embodiment of an amino acid sequence for dciAC. encoded by nucleotides 1983-2945 of the nucleic acid sequence shown in Figures 1A and 1B.
Figure 5 shows an embodiment of an amino acid sequence for dciAD, encoded by nucleotides 2950-3957 of the nucleic acid sequence shown In Figures 1A and 1B.
Figure 6 shows an embodiment of an amino acid sequence for dciAE, encoded by nucleotides. 3978-5609 of the nucleic acid sequence shown in Figures 1A and 1B, wherein the amino acids shown in underlined are omitted in an embodiment of inactive dciAA provided herein.

### Detailed Description of the Invention

The invention is as defined in the claims.

In one aspect of the invention, a method of increasing production and/or secretion of a polypeptide in a Bacillus cell is provided, wherein said cell selected for said method expresses at least one peptide transport protein according to the claims. Preferably, the cell selected endogenously expresses said peptide transport protein, however, the cell can be transformed to express said protein. The method comprises inactivating at least one peptide transport protein in said cell according to the claims. The method further comprises culturing said cell under conditions suitable for expression and secretion of said polypeptide.

"Peptide transport protein" as used herein refers to a protein involved in peptide transport. Proteins involved in peptide transport are considered to have peptide transport activity. A peptide transport protein includes a protein involved in dipeptide or oligopeptide transport, preferably dipeptide transport. Peptides which are transported by the peptide transport proteins or systems described herein include peptides which are 2-10 amino acids long, preferably 2-8, and more preferably 2-6. Oligopeptides may be 3-7 amino adds long, preferably 5 or 6 amino acids long and dipeptides are two amino acids.

The peptide transport protein which is inactivated is preferably involved in the import of proteins. As further discussed below, inactivation can occur in a variety of ways and by individual mutations or combinations of mutations. Peptide transport systems can include activity in exporting and/or importing peptides. In a preferred embodiment herein, peptide transport activity is interrupted. Preferably, importation of peptides by a peptide transport system is decreased or eliminated. Peptide transport proteins are known in the art and include proteins or gene products encoded by peptide transport operons as discussed below and known in the art.

An "operon" as used herein refers to a cluster of genes that are all controlled by the same promoter. A peptide transport operon includes at least one gene encoding a peptide transport protein. Peptide transport operons include the opp operon and the dciA operon and homologs thereof. According to the claims, a peptide transport operon excludes the opp operon and a peptide transport protein excludes proteins encoded by the opp operon.

The opp operon has been reported as encoding an oligopeptide permease that is required for the initiation of sporulation and the development of genetic competence (Rudner et al, 1991, Journal of Bacteriology, 173:1388-1398). The opp operon is a member of the family of ATP-binding cassette transporters involved in the import or export of oligopeptides from 3-5 amino acids. There are five gene products of the opp operon: oppA is the ligand-binding protein and is attached to the outside of the cell by a lipid anchor; oppB and oppC are the membrane proteins that form a complex through which the ligand is transported; oppD and oppF (Perego et al., 1991, Mol. Microbiol. 5:173-185) are the ATPases thought to provide energy for transport (LeDeaux, J. R., et al., 1997, FEMS Microbiology Letters 153: 63-69). The opp operon has also been referred to as SpoOK by Rudner et al., 1991, J. Bacteriol. 173:1388-1398.

Opp operons are also disclosed in Podbielski et al. 1996, Molecular Microbiology 21:1087-1099 and Tynkkynen et al. 1993, Journal of Bacteriology 175: 7523-7532. One assay for the presence or absence of a functioning opp operon is to subject the host to growth in the presence of toxic oligopeptide of 3 amino acids, such as Bialaphos, a tripeptide consisting of two L-alanine molecules and an L-glutamic acid analogue (Meijl Seika, Japan). A cell having a functional opp operon will have inhibited growth. A cell having a mutation in at least one gene of the opp operon gene cluster will not show growth inhibition in the presence of the toxic oligopeptide.

Further regarding peptide transport operons and peptides thereof, opp A,B,C,D, F of *Salmonella typhimurium* are further reported on in Hiles et al., 1987 J. Mol. Biol 195:125-142. OppA, B, C, D, F of *Bacillus subtilis* are further reported on in Perego, M., et al. Mol Microbiol 1991, 5, 173-185 and Rudner, D. Z., et al., J. Bacteriol 1991, 173(4):1388-1398. OppA, B, C, D, E of *E. coli* are reported on in Guyer, et al., 1985 J. Biol Chem 260:10812-10816. Also, a report has been made on amiA of *Streptococcus pneumonia* (Alloing, G 1994, J Mol Biol 241(1) :44-58. AppA, B, C, D, E of *B. subtilis* are reported on in Koide A. et al Mol Microbiol 1994 13(3) :417-426. Additionally, dppA of *E. coli* and *S. typhimurium* is reported on in Abouhamad et al 1991 Mol Microbiol 5(5):1035-1047. Furthermore, BldA, B, C, D, E of *Streptomyces coelicolor* are reported on in Nodwell, J.R. et al Mol Microbiol 1996, 22(5):881-93. OppA of *Streptococcus* is reported on in Podbielski, A et al Mol Microbiol 1996, 21 (5) :1087-1099. Moreover, TppA, B, C, D, E of *S. typhimurium* have been reported on in Gibson, M.M. et al., J. Bacteriol 1984 160:122-130. Moreover, it has been reported that oppA may also be obtained from *Chlamydia pneumonia.*

Organisms which a peptide transport operon or protein thereof can be obtained from include but are not limited to: *Aquifex aeolicus, Archaeoglobus fulgidus, Aeropyrum pernix, Bordetella pertussis, Bacillus subtilis, Clostridium acetobutylicum, Campylobacter jejuni, Chlorobium tepidum, Chlamydia pneumoniae CWL029, Chlamydia trachomatis Serovar D, Clostridium difficile, Corynebacterium diphtheriae, Deinococus radiodurans, Escherichia coli, Enterococcus faecalis, Haemophilus influenzae, Helicobacter pylori, Klebsiella pneumoniae, Mycobacterium leprae, Pseudomonas aeruginosa, Pyrococcus furiosus, Pyrococcus horikoshii, Pyrococcus abysii, Rhodobacter capsulatus, Streptococcus pyogenes* and *Salmonella typhimurium*.

The dciA operon has been reported on, see, e.g., Slack, et al., Mol Microbio (1991) 5(8), 1915-1925 and Mathlopoulos, et al., Mol Microbio (1991) 5(8), 1903-1913, as being a dipeptide transport operon in Bacillus. While this operon has been reported on, the function of each gene product has not previously been well characterized. Herein, provided are functions of the proteins of the dciA operon, including functional properties wherein a gene product is inactivated.

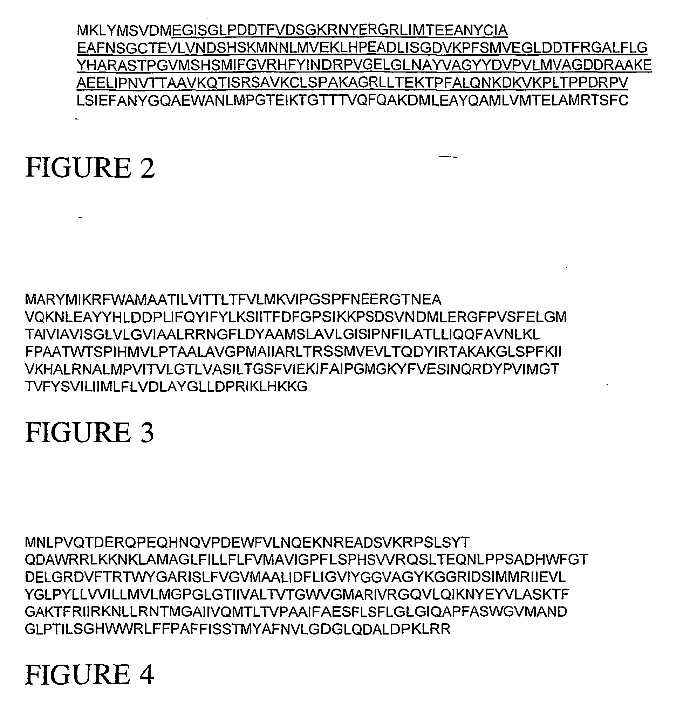

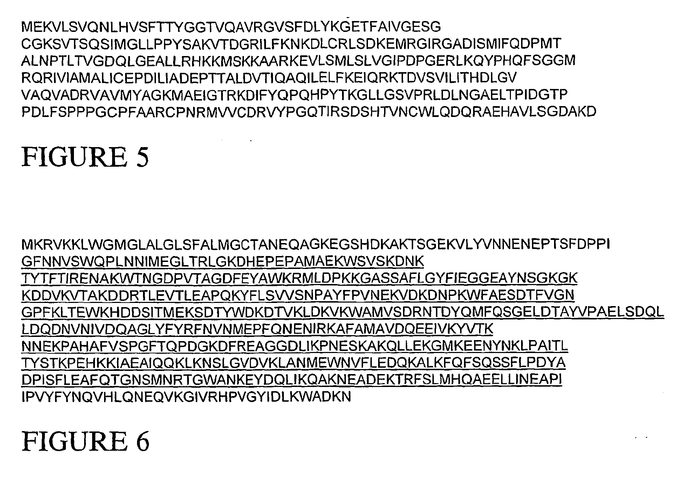

In one embodiment, dciAA is a peptide transport protein and inactivation of dciAA leads to increased polypeptide production and/or secretion. In another embodiment, inactivation of dciAE leads to decreased peptide transport and increased polypeptide production and/or secretion. In preferred embodiments, dciAA has RNA binding activity. Embodiments of dciAA, dciAB, dciAC, dciAD and dciAE are shown in Figures 2-6, respectively. DciA has homology to dpp of *E. coli.* Olson, et al., J Bacteriol 173:234-244 (1991); Kawarabayasi,Y., Journal DNA Res. 5 (2), 55-76 (1998).

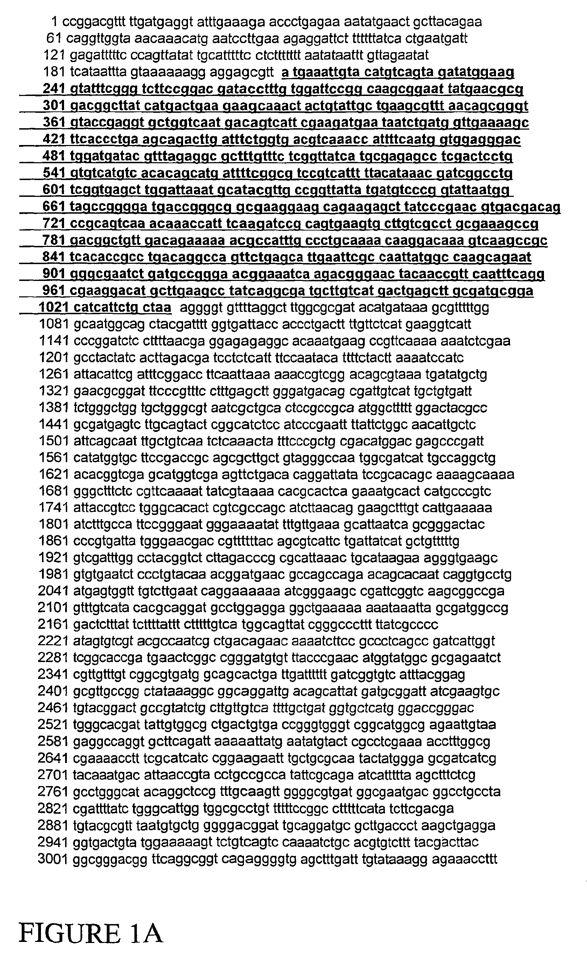

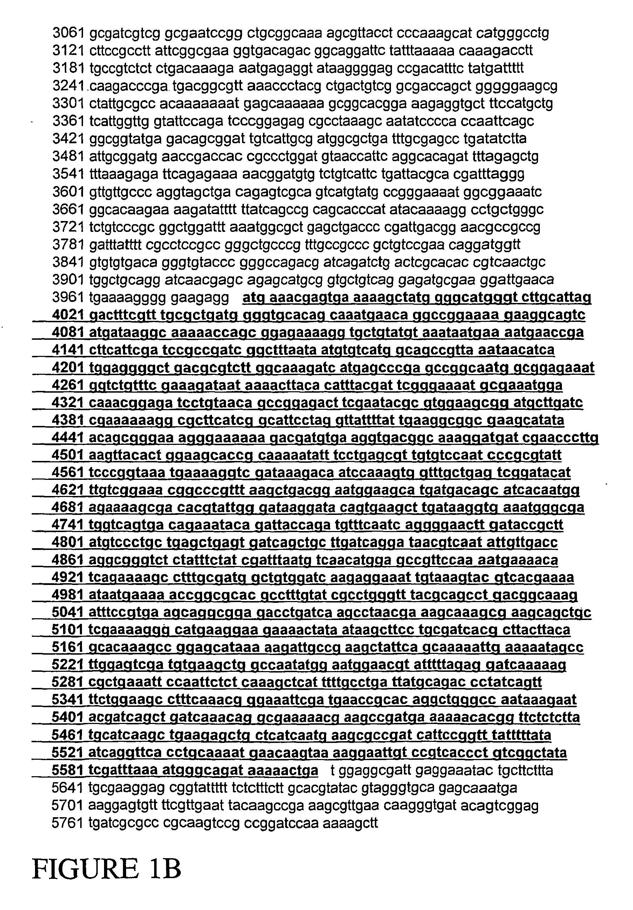

In addition to those described above, homologs of peptide transport operons, peptide transport genes and peptide transport proteins can be identified by a number of methods. In one embodiment, a nucleic acid is a "peptide transport gene" if it encodes a protein having peptide transport activity as discussed above. Preferably, the overall homology of the nucleic acid sequence is preferably greater than about 60%, more preferably greater than about 75%, more preferably greater than about 80%, even more preferably greater than about 85% and most preferably greater than 90% of one of the dciA genes shown in Figure 1, namely dciAA, dciAB, dciAC, dciAD or dciAE. In some embodiments the homology will be as high as about 93 to 95 or 98%.

In one embodiment, a protein is a "peptide transport protein" if it has peptide transport activity. Preferably the protein has overall homology greater than about 40%, more preferably greater than about 60%, more preferably at least 75%, more preferably greater than about 80%, even more preferably greater than about 85% and most preferably greater than 90% to the amino acid sequence of Figure 2, 3, 4, 5, or 6. In some embodiments the homology will be as high as about 93 to 95 or 98%.

Homology as used herein is in reference to sequence similarity or identity, with identity being preferred. This homology will be determined using standard techniques known in the art, including, but not limited to, the local homology algorithm of Smith & Waterman, Adv. Appl. Math. 2:482 (1981), by the homology alignment algorithm of Needleman & Wunsch, J. Mol. Biool. 48:443 (1970), by the search for similarity method of Pearson & Lipman, PNAS USA 85:2444 (1988), by computerized implementations of these algorithms (GAP, BESTFIT, FASTA, and TFASTA in the Wisconsin Genetics Software Package, Genetics Computer Group, 575 Science Drive, Madison, WI), the Best Fit sequence program described by Devereux et al., Nucl. Acid Res. 12:387-395 (1984), preferably using the default settings, or by inspection.

One example of a useful algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng & Doolittle, J. Mol. Evol. 35:351-360 (1987); the method is similar to that described by Higgins & Sharp CABIOS 5:151-153 (1989). Useful PILEUP parameters including a default gap weight of 3.00, a default gap length weight of 0.10, and weighted end gaps.

Another example of a useful algorithm is the BLAST algorithm, described in Altschul et al., J. Mol. Biol. 215, 403-410, (1990) and Karlin et al., PNAS USA 90:5873-5787 (1993). A particularly useful BLAST program is the WU-BLAST-2 program which was obtained from Altschul et al., Methods in Enzymology, 266: 460-480 (1996); http://blast.wustl/edu/blast/ REACRCE.html]. WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of Interest is being searched; however, the values may be adjusted to increase sensitivity. A % amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

Thus, "percent (%) nucleic acid sequence identity" is defined as the percentage of nucleotide residues in a candidate sequence that are identical with the nucleotide residues of the sequence shown in the nucleic acid figures. A preferred method utilizes the BLASTN module of WU-BLAST-2 set to the default parameters, with overlap span and overlap fraction set to 1 and 0.125, respectively.

The alignment may include the introduction of gaps in the sequences to be aligned. In addition, for sequences which contain either more or fewer nucleosides than those of the nucleic acid figures, it is understood that the percentage of homology will be determined based on the number of homologous nucleosides in relation to the total number of nucleosides. Thus, for example, homology of sequences shorter than those of the sequences identified herein and as discussed below, will be determined using the number of nucleosides in the shorter sequence.

In one embodiment, the peptide transport gene or operon is determined through hybridization studies. Thus, for example, nucleic acids which hybridize under high stringency to the nucleic acid sequences (that of the operon, the individual genes thereof or fragments thereof) identified in the figures, or a complement, are considered a peptide transport gene in one embodiment herein. High stringency conditions are known in the art; see for example Maniatis et al., Molecular Cloning: A Laboratory Manual, 2d Edition, 1989, and Short Protocols in Molecular Biology, ed. Ausubel, et al.. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology-Hybridization with Nucleic Acid Probes, "Overview of principles of hybridization and the strategy of nucleic acid assays" (1993). Generally, stringent conditions are selected to be about 5-10 C lower than the thermal melting point (Tm) for the specific sequence at a defined ionic strength pH. The Tm is the temperature (under defined ionic strength, pH and nucleic acid concentration) at which 50% of the probes complementary to the target hybridize to the target sequence at equilibrium (as the target sequences are present in excess, at Tm, 50% of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30 C for short probes (e.g. 10 to 50 nucleotides) and at least about 60 C for long probes (e.g. greater than 50 nucleotides). Stringent conditions may also be achieved with the addition of destabilizing agents such as formamide.

In another embodiment, less stringent hybridization conditions are used; for example, moderate or low stringency conditions may be used, as are known in the art; see Maniatis and Ausubel, supra, and Tijssen, supra.

Naturally occuring allelic variants of the genes and proteins provided herein may also be used in the methods of the present invention.

In addition to using the above techniques to find homologs of peptide transport genes, operons, proteins or peptide transport operon genes and proteins, one may use standard amplification of fragments of sequences as provided herein carried out in polymerase chain reaction (PCR) technologies such as described in Dieffenbach CW and GS Dveksler (1995, PCR Primer, a Laboratory Manual, Cold Spring Harbor Press, Plainview NY). A nucleic acid sequence of at least about 10 nucleotides and as many as about 60 nucleotides from an operon gene as provided herein, preferably about 12 to 30 nucleotides, and more preferably about 20-25 nucleotides can be used as a probe or PCR primer.

The term "nucleic acid" as used herein refers to deoxyribonucleotides or ribonucleotides and polymers thereof in either single- or double-stranded form. Unless specifically limited, the term encompasses nucleic acids containing known analogues of natural nucleotides which have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions) and complementary sequences and as well as the sequence explicitly indicated. Specifically, degenerate codon substitutions may be achieved by generating sequences in which the third position of one or more selected (or all) codons is substituted with mixed base and/or deoxyinosine residues (Batzer et al., Nucleic Acid Res. 19:5081 (1991); Ohtsuka et al., J. Biol. Chem. 260:605-2608 (1885); Cassol *et al*., 1992; Rossolini et al., Mol. Cell. Probes 8:81-98 (1994)). The term nucleic acid is used interchangeably with gene, cDNA, and mRNA encoded by a gene.

"Protein" as used herein includes proteins, polypeptides, and peptides. As will be appreciated by those in the art, the nucleic acid sequences of the invention can be used to generate protein sequences. Preferred peptide transport proteins have peptide transport activity prior to inactivation and/or are controlled by a peptide transport operon.

The methods provided herein are applicable for production or secretion of polypeptides in a cell type selected from a member of the family *Bacillus*.

In a preferred embodiments said cell is a member of the family *Bacillus* wherein said member of the family Bacillus is selected from the group consisting of *B. licheniformis, B. lentus, B. brevis, B. stearothermophilus, B. alkalophilus, B. amyloliquefaciens, B. coagulans, B. circulans, B. lautus, B. thuringiensis, B. methanolicus* and *B. anthracis*.

The polypeptide which is secreted or produced by the methods provided herein can be any polypeptide of interest. In a preferred embodiment, it is a heterologous polypeptide. Alternatively, the protein is homologous as discussed below.

In one aspect, the polypeptide to be produced and/or secreted is selected from the group consisting of hormone, enzyme, growth factor and cytokine. In one embodiment, the polypeptide is an enzyme. An enzyme as used herein includes but Is not limited to (i) oxidoreductases; (ii) transferases, comprising transferase transferring one-carbon groups (e.g., methyltransferases, hydroxymethyl-, formyl-, and related transferases, carboxyl- and carbamoyltransferases, amidinotransferases) transferases transferring aldehydic or ketonic residues, acyltransferases (e.g., acyltransferases, aminoacyltransferas), glycosyltransferases (e.g., hexosyltransferases, pentosyltransferases), transferases transferring alkyl or related groups, transferases transferring nitrogenous groups (e.g., aminotransferases, oximinotransferases), transferases transferring phosphorus-containing groups (e.g., phosphotransferases, pyrophosphotransferases, nucleotidyltransferases), transferases transferring sulfur-containing groups (e.g., sulfurtransferases, sulfotransferases, CoA-transferases), (iii) Hydrolases comprising hydrolases acting on ester bonds (e.g., carboxylic ester hydrolases, thioester hydrolases, phosphoric monoester hydrolases, phosphoric diester hydrolases, triphosphoric monoester hydrolases, sulfuric ester hydrolases), hydrolases acting on glycosyl compounds (e.g., glycoside hydrolases, hydrolyzing N-glycosyl compounds, hydrolyzing S-glycosyl compound), hydrolases acting on ether bonds (e.g., thioether hydrolases), hydrolases acting on peptide bonds (e.g., aminoacyl-peptide hydrolases, peptidyl-amino acid hydrolases, dipeptide hydrolases, peptidyl-peptide hydrolases), hydrolases acting on C-N bonds other than peptide bonds, hydrolases acting on acid-anhydride bonds, hydrolases acting on C-C bonds, hydrolases acting on halide bonds, hydrolases acting on P-N bonds, (iv) lyases comprising carbon-carbon lyases (e.g., carboxy-lyases, aldehyde-lyases, ketoacid-lyases), carbon-oxygen lyases (e.g., hydro-lyases, other carbon-oxygen lyases), carbon-nitrogen lyases (e.g., ammonia-lyases, amidine-lyases), carbon-sulfur lyases, carbon-halide lyases, other lyases, (v) isomerases comprising racemases and epimerases, cis-trans isomerases, intramolecular oxidoreductases, intramolecular transferases, intramolecular lyases, other isomerases, (vi) ligases or synthetases comprising ligases or synthetases forming C-O bonds, forming C-S bonds, forming C-N bonds, forming C-C bonds.

The polypeptide of interest may be a therapeutically significant protein, such as growth factors, cytokines, ligands, receptors and inhibitors, as well as vaccines and antibodies.

In a preferred embodiment, the precursor mRNA for the polypeptide to be produced or secreted includes a putative RNase cleavage site or a binding site for a peptide transport protein. In preferred embodiments, the polypeptide to be produced or secreted includes a specific peptide transport protein recognition site. Preferably the recognition site is a binding site. Preferably, the recognition site is a binding site for dciAA.

As used herein, the term "heterologous protein" refers to a protein or polypeptide that does not naturally occur in the host cell. The term "homologous protein" or "endogenously expressed" refers to a protein or polypeptide native or naturally occurring in the host cell. In one embodiment, the invention incudes host cells producing the homologous protein via recombinant DNA technology. A recombinant protein refers to any protein encoded by a nucleic acid which has been introduced into the host.

"Inactive protein" or grammatical equivalents as used herein refers to a reduction in the detectable activity of the protein when expressed in its wildtype form such as in its native unaltered host. Activities of peptide transport proteins include transport of dipeptides or oligopeptides of 3-7 amino acids, more preferably 5 or 6 amino acids, preferably in association with importation into the cell. In a preferred embodiment, peptide transport activity is reduced or eliminated.

In one aspect of the invention a gene product of a peptide transport operon is inactive or inactivated when the operon is activated. Preferably, the inactivated gene product is encoded by dciAA and/or dciAE. In a preferred embodiment, wildtype gene product corresponding to the inactive gene product comprises at least RNA binding or Rnase activity wherein the inactive gene product has decreased or eliminated interaction with RNA.

A peptide transport protein or a gene product of a peptide transport operon can be inactivated at the protein or nucleic acid level. It is understood that the cells and methods of the present invention may include more than one inactive protein. As described herein, two operons may be utilized, an opp operon and a dciA operon. For example, at least one gene product from an opp operon and at least one gene product from a dciA operon is inactive.

While a number of examples are discussed herein, it is understood that inactivation can occur by one or more mutations or modifications in a gene, protein or operon, or a combination thereof. For example, a mutation may be made in the oppA operon and one mutation may be made in the dciA operon, and more particularly in oppAA and dciAA. In Alternatively, 2 or more mutations are made to the same gene, protein or operon. Allernatively, 2 or more mutations are made wherein the mutations occur in different genes of the same operon, or in different operons, wherein the mutations can be in the same gene or different genes when the mutations are made in different operons. Generally, as used herein, mutation is used interchangeably with modification to refer to a change which infers inactivation of the gene, protein, system or activity as described herein.

In one aspect, the protein is inactivated because the gene encoding said protein has been mutated. The mutation can be caused in a variety of ways including one or more frameshifts, substitutions, insertions and/or deletions as further described below. The deletion can be of a single nucleotide or more, including deletion of the entire gene. It is understood that the cells comprising the inactive proteins described herein produced by methods of making said cells described wherein are each also provided herein.

In one embodiment, a cell having an inactive protein as described herein is arrived at by the replacement and/or inactivation of the naturally occurring gene from the genome of the host cell. In a preferred embodiment, the mutation is a non-reverting mutation.

One method for mutating nucleic acid encoding a gene is to clone the nucleic acid or part thereof, modify the nucleic acid by site directed mutagenesis and reintroduce the mutated nucleic acid into the cell on a plasmid. By homologous recombination, the mutated gene may be introduced into the chromosome. In the parent host cell, the result is that the naturally occurring nucleic acid and the mutated nucleic acid are located in tandem on the chromosome. After a second recombination, the modified sequence is left in the chromosome having thereby effectively introduced the mutation into the chromosomal gene for progeny of the parent host cell.

Another method for inactivating the gene product is through deleting the chromosomal gene copy. In a preferred embodiment, the entire gene is deleted, the deletion occurring in such as way as to make reversion impossible. In another preferred embodiment, a partial deletion is produced, provided that the nucleic acid sequence left in the chromosome is too short for homologous recombination with a plasmid encoded gene.

Deletion of the naturally occurring gene can be carried out as follows. A gene including its 5' and 3' regions is isolated and inserted into a cloning vector. The coding region of the gene is deleted form the vector *in vitro,* leaving behind a sufficient amount of the 5' and 3' flanking sequences to provide for homologous recombination with the naturally occurring gene in the parent host cell. The vector is then transformed into the host cell. The vector integrates into the chromosome via homologous recombination in the flanking regions. This method leads to a strain in which the gene has been deleted.

The vector used in an integration method is preferably a plasmid. A selectable marker may be included to allow for ease of identification of desired recombinant microorgansims. Additionally, as will be appreciated by one of skill in the art, the vector is preferably one which can be selectively integrated into the chromosome. This can be achieved by introducing an inducible origin of replication, for example, a temperature sensitive origin into the plasmid. By growing the transformants at a temperature to which the origin of replication is sensitive, the replication function of the plasmid is inactivated, thereby providing a means for selection of chromosomal integrants. Integrants may be selected for growth at high temperatures in the presence of the selectable marker, such as an antibiotic. Integration mechanisms are described in WO 88/06623.

Integration by the Campbell-type mechanism can take place in the 5' flanking region of the gene, resulting in a strain carrying the entire plasmid vector in the chromosome in the locus. Since illegitimate recombination will give different results it will be necessary to determine whether the complete gene has been deleted, such as through nucleic acid sequencing or restriction maps.

Another method of inactivating the naturally occurring gene is to mutagenize the chromosomal gene copy by transforming a cell with oligonucleotides which are mutagenic. Alternatively, the chromosomal protease gene can be replaced with a mutant gene by homologous recombination.

In a preferred embodiment, the present invention encompasses host cells having further protease deletions or mutations. For example, well know protease deletions in Bacillus, including deletions or mutations in apr, npr, epr, mpr generally used for efficient heterologous expression. Other embodiments of the protease are described in, for example, U.S. Patent No. 5,264,366.

Other ways of inactivating a protein at the nucleic acid level include the use of antisense molecules. Antisense or sense oligonucleotides, according to the present invention, comprise a fragment of the coding region of a peptide transport protein or a product of a peptide transport operon. Such a fragment generally comprises at least about 14 nucleotides, preferably from about 14 to 30 nucleotides. The ability to derive an antisense or a sense oligonucleotide, based upon a cDNA sequence encoding a given protein is described in, for example, Stein and Cohen (Cancer Res. 48:2659, 1988) and van der Krol et al. (BioTechniques 6:958, 1988). Binding of antisense or sense oligonucleotides to target nucleic acid sequences results in the formation of duplexes that block transcription or translation of the target sequence by one of several means, including enhanced degradation of the duplexes, premature termination of transcription or translation, or by other means.

Ribozymes may also be used for inactivation in one embodiment. Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, e.g., Rossi, Current Biology, 4:469-471 (1994), and PCT publication No. WO 97/33551 (published September 18, 1997).

The peptide transport protein or product of a peptide transport operon may also be inactive or inactivated at the protein level. For example, the nucleic acid encoding the protein or gene product may be intact, but the cell may comprise an antagonist or inhibitor such as an antibody to inhibit the protein or gene product from having its native activity, such as peptide transport or RNA interaction activity. Moreover, the protein may be expressed as an inactive variant or be conditionally inactive, for example, by having temperature sensitive peptide transport.

For production and/or secretion of proteins in a cell, an expression vector comprising at least one copy of a nucleic acid encoding the heterologous or homologous protein, and preferably comprising multiple copies, is transformed into the cell under conditions suitable for expression of the protein.

Expression vectors used in the present invention comprise at least one promoter associated with the protein, which promoter is functional in the host cell. In one embodiment of the present invention, the promoter is the wild-type promoter for the selected protein and in another embodiment of the present invention, the promoter is heterologous to the protein, but still functional in the host cell. In one preferred embodiment of the present invention, nucleic acid encoding the polypeptide of interest is stably integrated into the host genome. Signal sequences may be added if needed.

In a preferred embodiment, the expression vector contains a multiple cloning site cassette which preferably comprises at least one restriction endonuclease site unique to the vector, to facilitate ease of nucleic acid manipulation. In a preferred embodiment, the vector also comprises one or more selectable markers. As used herein, the term selectable marker refers to a gene capable of expression in the host which allows for ease of selection of those hosts containing the vector. Examples of such selectable markers include but are not limited to antibiotics, such as, erythromycin, actinomycin, chloramphenicol and tetracycline.

In one embodiment of the present invention, nucleic acid encoding at least one polypeptide of interest is introduced into a host cell via an expression vector capable of replicating within the host cell. Suitable replicating plasmids for *Bacillus* are described in Molecular Biological Methods for Bacillus, Ed. Harwood and Cutting, John Wiley & Sons, 1990, hereby expressly incorporated by reference; see chapter 3 on plasmids. Suitable replicating plasmids for *B. subtilis* are listed on page 92. Several strategies have been described in the literature for the direct cloning of DNA in *Bacillus.* Plasmid marker rescue transformation involves the uptake of a donor plasmid by competent cells carrying a partially homologous resident plasmid (Contente et al., Plasmid 2:555-571 (1979); Haima et al., Mol. Gen. Genet. 223:185-191 (1990); Weinrauch et al., J. Bacteriol. 154(3):1077-1087 (1983); and Weinrauch et al., J. Bacteriol. 169(3):1205-1211 (1987)). The incoming donor plasmid recombines with the homologous region of the resident "helper" plasmid in a process that mimics chromosomal transformation.

Transformation by protoplast transformation is described for *B. subtilis* in Chang and Cohen, (1979) Mol. Gen. Genet 168:111-115; for B.megaterium in Vorobjeva et al., (1980) FEMS Microbiol. Letters 7:261-263; for B. amyloliquefaciens in Smith et al., (1986) Appl. and Env. Microbiol. 51:634; for B.thuringiensis in Fisher et al., (1981) Arch. Microbiol. 139:213-217; for B.sphaericus in McDonald (1984) J. Gen. Microbiol. 130:203; and B.larvae in Bakhiet et al., (1985) 49:577. Mann et al., (1986, Current Microbiol. 13:131-135) report on transformation of *Bacillus* protoplasts and Holubova, (1985) Folia Microbiol. 30:97) disclose methods for introducing DNA into protoplasts using DNA containing liposomes. The presence/absence of a marker gene can suggest whether the gene of interest is present in the host cell.

Alternatively, host cells which contain the coding sequence for the polypeptide of interest may be identified by a variety of procedures known to those of skill in the art. These procedures include, but are not limited to, DNA-DNA or DNA-RNA hybridization and protein bioassay or immunoassay techniques which include membrane-based, solution-based, or chip-based technologies for the detection and/or quantification of the nucleic acid or protein.

There are various assays known to those of skill in the art for detecting and measuring activity of secreted polypeptides. In particular, for proteases, there are assays based upon the release of acid-soluble peptides from casein or hemoglobin measured as absorbance at 280 nm or colorimetrically using the Folin method (Bergmeyer, et al., 1984, Methods of Enzymatic Analysis vol. 5, Peptidases, Proteinases and their Inhibitors, Verlag Chemie, Weinheim). Other assays involve the solubilization of chromogenic substrates (Ward, 1983, Proteinases, in Microbial Enzymes and Biotechnology (W.M. Fogarty, ed.), Applied Science, London, pp. 251-317).

Means for determining the levels of secretion of a heterologous or homologous protein in a host cell and detecting secreted proteins include, using either polyclonal or monoclonal antibodies specific for the protein. Examples include enzyme-linked immunosorbent assay (ELISA), radioimmunoassay (RIA) and fluorescent activated cell sorting (FACS). These and other assays are described, among other places, in Hampton R et al (1990, Serological Methods, a Laboratory Manual, APS Press, St Paul MN) and Maddox DE et al (1983, J Exp Med 158:1211). In a preferred embodiment, secretion is higher using the methods and compositions provided herein than when using the same methods or compositions, but where a peptide transport protein or gene product of a peptide transport operon has not been inactivated. In a preferred embodiment, wherein RNase activity is decreased, production and/or secretion of polypeptides is increased. In another preferred embodiment, wherein peptide transport activity is decreased, production and/or secretion is increased.

A wide variety of labels and conjugation techniques are known by those skilled in the art and can be used in various nucleic and amino acid assays. Means for producing labeled hybridization or PCR probes for detecting specific polynucleotide sequences include oligolabeling, nick translation, end-labeling or PCR amplification using a labeled nucleotide. Alternatively, the nucleotide sequence, or any portion of it, may be cloned into a vector for the production of an mRNA probe. Such vectors are known in the art, are commercially available, and may be used to synthesize RNA probes *in vitro* by addition of an appropriate RNA polymerase such as T7, T3 or SP6 and labeled nucleotides.

A number of companies such as Pharmacia Biotech (Piscataway NJ), Promega (Madison WI), and US Biochemical Corp (Cleveland OH) supply commercial kits and protocols for these procedures. Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US Patents 3,817,837; 3,850,752; 3,939,350; 3,996,345; 4,277,437; 4,275,149 and 4,366,241. Also, recombinant immunoglobulins may be produced as shown in US Patent No. 4,816,567 and incorporated herein by reference.

The cells transformed with polynucleotide sequences encoding heterologous or homologous protein or endogenously having said protein may be cultured under conditions suitable for the expression and recovery of the encoded protein from cell culture. Other recombinant constructions may join the heterologous or homologous polynucleotide sequences to nucleotide sequence encoding a polypeptide domain which will facilitate purification of soluble proteins (Kroll DJ et al (1993) DNA Cell Biol 12:441-53).

Such purification facilitating domains include, but are not limited to, metal chelating peptides such as histidine-tryptophan modules that allow purification on immobilized metals (Porath J (1992) Protein Expr Purif 3:263-281), protein A domains that allow purification on immobilized immunoglobulin, and the domain utilized in the FLAGS extension/affinity purification system (Immunex Corp, Seattle WA). The inclusion of a cleavable linker sequence such as Factor XA or enterokinase (Invitrogen, San Diego CA) between the purification domain and the heterologous protein can be used to facilitate purification.

The manner and method of carrying out the present invention may be more fully understood by those of skill in the art by reference to the following examples.

### Example 1

Example I illustrates the increase in production of subtilisin and amylase from B. subtilis having a mutation in the dciAE gene of the dciA operon.

### Production of subtilisin from strains containing a dciAE wild type or a dciAE mutant in shake flasks.

Strains to be tested were grown in shake flasks containing 25 ml of LB (Difco) in a 250 mL flask. Shake flasks were incubated at 37°C with vigorous shaking and at OD 550 of 0.8, 1 mL of culture were mixed with 0.5 ml 30 % Glycerol and frozen for further experiments. 30 ul of the thawed vials were used to inoculate 40 ml of a media containing 68 g/L Soytone, 300 M PIPES, 20 g/L Glucose (final pH 6.8) in 250 mL flasks. The shake flasks are incubated at 37°C with vigorous shaking for three days, after which they are sampled for subtilisin analysis of the supernatant.

Supernatants from liquid cultures were harvested after different times during growth and assayed for subtilisin as previously described (Estell, D. V., Graycar, T. P., Wells, J. A. (1985) J. Bio/. Chem. 260, 6518-6521) in a solution containing 0.3 mM *N*-succinyl-L-Ala-L-Ala-L-Pro-L-Phe-p-nitroanalide (Vega Biochemicals), 0.1 M Tris, pH 8.6, at 25°C. The assays measured the increase in absorbance at 410 nm/min due to hydrolysis and release of *p*-nitroanaline. Table 1 describes the yields of protease produced from the two strains tested.

After 24 hours the strain deleted for dciAE secreted 3.5 times more subtilisin than the control. After 48 hours the increase is 4.9 times (Table 1).

**Table 1**

| B. subtilis strains | Genotype | Subtilisin (rate) 48 h |
|---|---|---|
| 2790 | dciAE wt | 0.888 |
| 2790 | dciAE- | 4.38 |

### Production of amylase from strains containing an dciAE wild type or an dciAE mutant in shake flasks.

Strains to be tested were grown in shake flasks containing 25 ml of LB (Difco) in a 250 mL flask. Shake flasks were incubated at 37°C with vigorous shaking and at OD 550 of 0.8, 1 mL of culture were mixed with 0.5 ml 30 % Glycerol and frozen for further experiments. 30 ul of the thawed vials were used to inoculate 40 ml of a media containing 68 g/L Soytone, 300 M PIPES, 20 g/L Glucose (final pH 6.8) in 250 mL flasks. The shake flasks are incubated at 37°C with vigorous shaking for several days during which they are sampled for amylase analysis of the supernatant.

Whole broth samples were spun down at different times of growth and their supernatants were assayed as follows. Supernatant is mixed in a cuvete with 790.0 ul of substrate (Megazyme-Ceralpha-Alpha Amylase; substrate is diluted in water and is used as 1 part substrate plus 3 parts of Alpha Amylase buffer pH 6.6) at 250C. Alpha Amylase buffer is composed of 50mM Maleate Buffer, 5 mM CaCl2, and 0.002 % Triton X-100, PH= 6.7. Amylase was measured in a Spectronic Genesys 2 Spectophotometer using a protocol for amylase activity (Wavelenth: 410 nm, Initial Delay: 75 secs., Total Run Time: 120 secs, Lower Limit: 0.08, Upper Limit: 0.12).

Results show that the strain containing the dciAE deletion produced 1.8 times more amylase at 48 hours than when the dciAE wild-type gene was present. At 60 hours the increase is 2.5 times (Table 2).

Table 2 describes the yields of amylase produced from the two strains tested.

**Table 2**

| ***B. subtilis* strains** | **Genotype** | **Amylase (rate) 48 h** | **60h** |
|---|---|---|---|
| 2790 | dciAE wt | 0.054 | 0.066 |
| 2790 | dciAE- | 0.098 | 0.168 |

### EXAMPLE 2

Example 2 illustrates the additional increase of production of subtilisin from B. subtilis having a mutation in the oppAA of the oppA operon and a deletion of dciAA gene of the dciA operon.

### Production of recombinant subtilisin from strains containing a dciAA deletion and an oppAA mutation in shake flasks.

Strains to be tested were grown in shake flasks containing 25 ml of LB (Difco) and 25 ug/L chloramphenicol in a 250 ml flasks. Shake flasks were incubated at 37 C with vigorous shaking and at OD 550 of 0.8, 1 ml of culture were mixed with 0.5 ml 30 % Glycerol and frozen for further experiments. 25 ml of FNA34EB media (Per liter: 90.72 g PIPES acid form, pH to 6.8, 34.00 g Soytone (Difco), 2 drops of Mazu DF-204, 40 mL 50% glucose) in 250 ml flasks.

10 uL of glycerol stocks from the strains to be tested was added to each flask. Once inoculated, the flasks were incubated at 37 degrees C, 250 rpm and samples were taken every 24 hours until 75 hours. 1-mL samples were taken out in the sterile hood and measured for OD 550. Samples were centrifuged for 1 minute to pellet cells.

Supernatants from liquid cultures were harvested after different times during growth and assayed for subtilisin as previously described (Estell, D. V., Graycar, T. P., Wells, J. A. (1985) J. Biol. Chem. 260, 6518-6521) in a solution containing 0.3 mM *N-*succinyl-L-Ala-L-Ala-L-Pro-L-Phe-p-nitroanalide (Vega Biochemicals), 0.1 M Tris, pH 8.6, at 25°C. The assays measured the increase in absorbance at 410 nm/min due to hydrolysis and release of *p-*nitroanaline.

Table 3 describes the yields of protease produced from the two strains. Results show that the strain containing the dciAA deletion and the oppAA mutation produced 1.9 more protease than when the dciAA wild-type was present.

**Table 3**

| B. subtilis strains | Genotype | Subtilisin (mg/L) | | |
|---|---|---|---|---|
| | | 27h | 51h | 75h |
| 001 | oppAA- | 30 | 105 | 138 |
| 040 | oppAA- dciAA- | 28 | 124 | 262 |

The effect found deleting the dciAA protein in increase enzyme production could be due to the possible interaction between this protein and the subtilisin (apr) mRNA. As demonstrated in the next section we show there is some homology between dciAA and proteins that interact with the RNA, furthermore, we show that we found fact that there is a "putative Rnase cleavage site" in the untranslated mRNA region of subtilisin (starting in the +1 mRNA). This putative RnaseE cleavage site in subtilisin has 6/7 identity with a site identified in RNA I (table 4). This site is cleavaged by RNAase E (Tomcsanyi and Cohen S 1985; Lin-Chao and Cohen S 1991; Lin-Chao et al 1994). The putative Rnase E site has 7/7 identity with the demonstrated cleavage site for RnaseE in 9S RNA (Roy, M. K., and Apirion, D., Biochim. Biophys. Acta 747, 200-208 1983) (Table 4).

**Table 4**

| RNA | location | Sequence |
|---|---|---|
| Subtilisin | +1 5' mRNA | ACAGAAU |
| RNA I | +1 5' mRNA | ACAGUAU |
| 9S RNA | +1 5' mRNA | ACAGAAU |

Without being bound to theory, we believe that it could be possible that one of the functions of the dciAA protein is binding to this region of the RNA affecting the stability of this RNA or the translation of the protein. Thus, also provided herein is a method of increasing peptide production/secretion of proteins that contain a putative dciAA recognition site.

### Homology between dciAA and other proteins.

The predicted product of the dciAA gene was used to search a translation of the GeneBank data base and blast homology was found between the dciAA gene product and the dppA protein from B. methanolicus (75% identity), a putative peptide ABC transporter from Deinocuccus radiodurans (33% identity), and with the dppA dipeptide transport protein from Pyrococcus abyssi (28%). The search program used was BSORF by fasta 3 t and blast. Several dipeptide proteins from *B. methanolicus* 75% shows identity with a putative transport associated protein in Streptomyces coelicolor, 32 % identity with a dipeptide transport protein dppA from *Pyrococcus abyssi*.

By doing the search using different regions of the dciAA protein in the GeneBank database (BSORF by fasta 3 t and blast) homology was found between the dciAA gene product with a ribonuclease from E. coli (Rnase E aka Ams) (Claverie-Martin et al J. Biol. Chem. 266: 2843-2851, 1991). Both proteins shows a 25% identity (and 48% conservative substitutions) over a region of 47 amino acids in the carboxy-terminal portions of both proteins. DciAA also share 26% identity with the carboxy-terminal portion of ribonucleoprotein La from Homo sapiens (Chan et al Nucleic Acid Research 17, 2233-2244 ,1989).

### Homology Between dciAE and Other Proteins.

The predicted product of the dciAE was used to search a translation of the GeneBank data base. The search program used was BSORF by festa 3t and blast. Homology was formed within OPPA oligopeptide-binding protein OppA of B. subtilis (40% identity) pX02-66 of B. anthracis (32% identity), Trac of Enterococcus faecalis (30% identity), MppA periplasmic murein peptide-binding protein precursor of E. coli (30% identity), OppA-Salty periplasmic oligopeptide-binding protein (oligopeptide-binding protein precursor) of Salmonella typhimurium (30% identity), oligopeptide permease homolog All (Borrelia burgdorferi) (29% identity), oligopeptide ABC transporter, periplasmic oligopeptide-binding protein (OppAV) homolog-lyme disease spirochete Borrelia burgdorferi), oligopeptide-binding protein from Chlamydophila pneumonia (26% identity), and to some extent, oligopeptide ABC transporter periplasmic binding protein OppA-syphilis spirochaete from Treponema pallidum (26% identity).

### Deletion of the dipeptide transport system dciAA gene.

Using the PCR technique, 647 bp of the dciAA gene present in strains 001 and BG2790 was deleted. Two amplified DNA fragments containing part of the 5' (using primers dci1.f and dci3.3) and 3' of the gene (using primers dci2.r and dci4.f) were ligated and cloned in a pTSpUC19Kan plasmid.

Plasmid pTSpUC19Kan carries a Kanamycin resistance gene (Kanr) and a temperature sensitive origin of replication (TsOri). The dciAA disruption plasmid, pMdci, was protoplast transformed into the two strains. Because of the TsOri, this plasmid integrated into the chromosome at the region of homology with the dciAA gene when cultured under selective pressure at the non-permissive temperature, e.g. 48 C. After integration, strains carrying the integrated plasmid were grown extensively at permissive temperature. Upon excision of the integrated plasmid, either the parent strain 001 or BG2790 is restored, or strains carrying the deleted gene is constructed. Two new strains were confirmed by PCR amplification of the gene with primers based in the gene sequence to contain the deletion of the gene.

### Deletion of the dipeptide transport system dciAE gene.

Using the PCR technique, 1228 bp of the dciAE gene present in strain BG2970 was deleted. Two amplified DNA fragments containing part of the 5' (using primers dciAE1 and dciAE2) and 3' of the gene (using primers dciAE3 and dciAE4) were ligated and cloned in a pTSpUC19Kan plasmid. Plasmid pTSpUC19Kan carries a Kanamycin resistance gene (Kanr) and a temperature sensitive origin of replication (TsOri). The dciAE disruption plasmid, pMdciE, was protoplast transformed into the BG2790 production strain . Because of the TsOri, this plasmid integrated into the chromosome at the region of homology with the dciAE gene when cultured under selective pressure at the non-permissive temperature, e.g. 48 C. After integration, three strains carrying the integrated plasmid were grown extensively at permissive temperature. Upon excision of the integrated plasmid, either the parent strain BG2790 is restored, or a strain carrying the deleted gene is constructed. The new strain was confirmed by PCR amplification of the gene with primers based in the gene sequence to contain the deletion of the gene.

### Primers:

dci1.f 
GCGCGCGGATCCCGTCTGAATGAATTGTTATCGGTTTTCAGCCGTGTAC
GGG
dci2.r 
GCGCGCCTGCAGCGGGATGGAGATGCCGAGTACTGCAAGACTCATCGC
GGCG
dci3.r
   GCGCGCGTCGACCCATATCTACTGACATGTACAATTTCATAACGC
dci4.f
   GCGCGCGTCGACGCCGCTCACACCGCCTGACAGGCCAGTTCTGAGC
dciAE1
   GCGCGCGGATCCGATGTGTCTGTCATTCTGATTACGC
dciAE2
   GCGCGCGTCGACGATCGGCGGATCGAATGAAGTCGG
dciAE3
   GCGCGCGTCGACCCAATAAAGAATACGATCAGCTGATC
dciAE4
   GCGCGCCTGCAGTGTCCCAAAACCCCCGATGCGCAC

### EXAMPLE 3

Example 3 illustrates a method for screening for microorganisms with increased production of proteins, preferably secreted proteins. More particularly, the method is for screening for cells having interrupted peptide transport activity, particularly an inhibition in import of peptides, which results in an increase in production of polypeptides. The method involves subjecting cells to a toxic peptide which is the size of a peptide which would normally be imported into the cell by the peptide transport system. The toxic peptide may be Bialaphos. Bislaphos is a tripeptide consisting of two L-alanine molecules and an L-glutamic acid analogue.
When digested by peptidases it is deleterious to the cell. It is an antibiotic, see, Meiji Seika, Japan.

There has been reported in the literature that at least one operon in Bacillus is involved in the uptake of molecules of the size of Bialaphos (3-amino acids), the oppA operon. There may also be other additional genes involved in Bialaphos uptake.

The method allows an efficient method for screening for cells which have interrupted peptide transport and increased production of polypeptides. Specifically, the method screens for mutation(s) which result in decreased peptide uptake. Cells which survive exposure to Bialaphos are identified as having one or more such mutations. The method does not require further characterization of the mutation, for example, at the nucleic acid level, thereby providing efficiency. However, further screening can occur such as for increased production of polypeptides, and further characterization and optimization of the mutation(s). Exposure to Bialaphos can occur in a number of ways such as plating cells with Bialaphos, for example, 50 ug/ml.

The interrupted peptide transport can occur because the strain is mutated, or because the cell has been mutated, or because genes, proteins or operons have been mutated or modified so as to result in increased polypeptide production as described herein. Any one or all of the genes in one or more peptide transport operons may be mutated or modified.

A microorganism or cell having a functional peptide transport operon, for example, the opp operon, will have inhibited growth. A microorganism having a mutation in at least one gene of the opp operon gene cluster will not show growth inhibition in the presence of the toxic oligopeptide.

### Method to obtain Bialaphos resistant mutants.

Cells are grown in liquid culture (LB) until they reach exponential phase. Cultures are plated onto Bacillus minimal Agar plates containing different concentrations of Bialaphos. Plates are incubated overnight at 37°C and growing colonies are streaked out for purification on same type of plates.

These new strains are Bialaphos resistance and all produce more protease than the parental strain after overnight growth.

### Protease assay.

Supernatants from liquid culture were harvested after 3 days of growth and assayed for subtilisin as previously described (Estell, D.V., Graycar, T.P., Wells, J.A. (1985) J. Biol Chem. 260:6518-6521) in a solution containing 03 mM *N-*succinyl-L-Ala-L-Ala-L-Pro-L-Phe-p-nitroanalide (Vega Biochemicals), 0.1 M Tris, pH 8.6, at 25°C. The assays measured the increase in absorbance at 410 nm/min due to hydrolysis and release of *p*-nitroanaline. The results were easily detectable and confirmed that one can identify cells having increased protein production by screening for cells which do not uptake peptides. In one embodiment, mutations to oppA are excluded. In another embodiment, Bacillus is excluded as an organism to screen.

## Claims

1. A method of increasing secretion of a polypeptide in a *Bacillus* cell wherein said cell expresses a dciAE dipeptide transport protein of a dciA dipeptide transport operon comprising:
inactivating said dciAE dipeptide transport protein in said cell, ; and
culturing said cell under conditions suitable for expression and secretion of said polypeptide.

2. The method of claim 1, wherein said inactivating comprises mutating a nucleic acid comprising nucleic acid encoding a gene product of said dciA operon.

3. The method of claim 1 or 2, wherein said inactivating comprises mutating a nucleic acid comprising nucleic acid encoding dciAE or dciAA.

4. The method of claim 2 or 3, wherein said mutating causes a frameshift or is deleting one or more nucleotides.

5. The method of any one of the preceding claims, wherein said *Bacillus* cell is selected from the group consisting of *B. licheniformis, B. lentus, B. brevis, B. stearathermophilus, B. alkalophilus, B. amyloliquefaciens, B. coagulans, B. circulans, B. lautus, B. methanolicus, B. anthracis* and *B. thuringiensis*.

6. The method of any one of claims 1 to 4, wherein said *Bacillus* cell is *B. subtilis*.

7. The method of any one of the preceding claims, wherein said polypeptide is selected from the group consisting of hormone, enzyme, growth factor and cytokine.

8. The method of any one of the preceding claims, wherein said polypeptide is heterologous.

9. The method of any one of the preceding claims, wherein said polypeptide is an enzyme selected from the group consisting of proteases, carbohydrates, reductases, lipases, isomerases, transferases, kinases phophatases, cellulase, endoglucosidase H, oxidase, alpha-amylase, lignocellulose hemicellulase, pectinase, and liginase.

10. The method of any one of the preceding claims, wherein said polypeptide is a bacillus protease, a subtilisin, an amylase, or a bacillus amylase.

11. A method for producing a polypeptide in a *Bacillus* cell comprising the steps of:
(a) obtaining a *Bacillus* cell comprising nucleic acid encoding a polypeptide to be produced, said cell further comprising a dciA dipeptide transport operon, wherein at least one gene product of said operon is inactive in said cell; and
(b) culturing said cell under conditions suitable for expression such that said polypeptide is produced.

12. The method of claim 11, wherein said gene product is inactive as the result of mutation in said operon.

13. The method of claim 12, wherein said mutation is a frameshift mutation or is a deletion of one or more nucleotides.

14. The method of any one of claims 11 to 13, wherein said inactive gene product is dciAE or dciAA.

15. The method of any one of claims 11 to 14, wherein said polypeptide is heterologous.

16. A method of producing a *Bacillus* cell having increased polypeptide secretion, comprising inactivating a dciA dipeptide transport operon in said cell.

17. The method of claim 16, wherein said operon has been mutated to inactivate a gene product of a dciAE gene or a dciAA gene.

## Patentansprüche

1. Verfahren zur Erhöhung der Sekretion eines Polypeptids in einer *Bacillus*-Zelle, worin die Zelle ein dciAE-Dipeptid-Transportprotein eines dciA-Dipeptid-Transportoperons exprimiert, umfassend:
das Deaktivieren des dciAE-Dipeptid-Transportproteins in der Zelle; sowie
das Züchten der Zelle unter Bedingungen, die für die Expression und Sekretion des Polypeptids geeignet sind.

2. Verfahren nach Anspruch 1, worin das Deaktivieren das Mutieren einer Nucleinsäure umfasst, die Nucleinsäure umfasst, die für ein Genprodukt des dciA-Operons kodiert.

3. Verfahren nach Anspruch 1 oder 2, worin das Deaktivieren das Mutieren einer Nucleinsäure umfasst, die Nucleinsäure umfasst, die für dciAE oder dciAA kodiert.

4. Verfahren nach Anspruch 2 oder 3, worin das Mutieren eine Rasterverschiebung bewirkt oder ein oder mehrere Nucleotide deletiert.

5. Verfahren nach einem der vorangegangenen Ansprüche, worin die *Bacillus-*Zelle aus der aus *B. licheniformis, B. lentus, B. brevis, B. stearothermophilus, B. alkalophilus, B. amyloliquefaciens, B. coagulans, B. ciruculans, B. lautus, B. methanolicus, B. anthracis* und *B. thuringiensis* bestehenden Gruppe ausgewählt ist.

6. Verfahren nach einem der Ansprüche 1 bis 4, worin die *Bacillus-Zelle B. subtilis* ist.

7. Verfahren nach einem der vorangegangenen Ansprüche, worin das Polypeptid aus der aus Hormonen, Enzymen, Wachstumsfaktor und Zytokin bestehenden Gruppe ausgewählt ist.

8. Verfahren nach einem der vorangegangenen Ansprüche, worin das Polypeptid heterolog ist.

9. Verfahren nach einem der vorangegangenen Ansprüche, worin das Polypeptid ein Enzym ist, das aus der aus Proteasen, Kohlenhydraten, Reduktasen, Lipasen, Isomerasen, Transferasen, Kinasen, Phosphatasen, Cellulasen, Endoglucosidase-H, Oxidase, α-Amylase, Lignocellulose, Hemicellulase, Pectinase und Liginase bestehenden Gruppe ausgewählt ist.

10. Verfahren nach einem der vorangegangenen Ansprüche, worin das Polypeptid eine *Bacillus*-Protease, ein Subtilisin, eine Amylase oder eine Bacillus-Amylase ist.

11. Verfahren zur Produktion eines Polypeptids in einer Bacillus-Zelle, umfassend folgende Schritte:
(a) das Erhalten einer Bacillus-Zelle, die Nucleinsäure umfasst, die für ein zu produzierendes Polypeptid kodiert, wobei die Zelle weiters ein dciA-Dipeptid-Transportoperon umfasst, worin zumindest ein Genprodukt des Operons in der Zelle deaktiviert ist; sowie
(b) das Züchten der Zelle unter Bedingungen, die für die Expression geeignet sind, so dass das Polypeptid produziert wird.

12. Verfahren nach Anspruch 11, worin das Genprodukt als Resultat einer Mutation in dem Operon inaktiv ist.

13. Verfahren nach Anspruch 12, worin die Mutation eine Rasterverschiebungsmutation ist oder eine Deletion eines oder mehrerer Nucleotide ist.

14. Verfahren nach einem der Ansprüche 11 bis 13, worin das inaktive Genprodukt dciAE oder dciAA ist.

15. Verfahren nach einem der Ansprüche 11 bis 14, worin das Polypeptid heterolog ist.

16. Verfahren zur Produktion einer Bacillus-Zelle mit erhöhter Polypeptid-Sekretion, umfassend das Deaktivieren eines dciA-Dipeptid-Transportoperons in der Zelle.

17. Verfahren nach Anspruch 16, worin das Operon mutiert worden ist, um ein Genprodukt eines dciAE-Gens oder eines dciAA-Gens zu deaktivieren.

## Revendications

1. Méthode pour augmenter la sécrétion d'un polypeptide dans une cellule de *Bacillus,* où ladite cellule exprime une protéine de transport de dciAE dipeptide d'un opéron de transport de dciA dipeptide comprenant:
inactiver ladite protéine de transport de dciAE dipeptide dans ladite cellule; et
cultiver ladite cellule sous des conditions qui conviennent pour l'expression et la sécrétion dudit polypeptide.

2. Méthode selon la revendication 1, dans laquelle ladite inactivation comprend la mutation d'un acide nucléique comprenant l'acide nucléique codant pour un produit de gène dudit opéron dciA.

3. Méthode selon la revendication 1 ou 2, où ladite inactivation comprend la mutation d'un acide nucléique comprenant l'acide nucléique codant pour dciAE ou dciAA.

4. Méthode selon la revendication 2 ou 3, dans laquelle ladite mutation provoque un changement de phase ou efface un ou plusieurs nucléotides.

5. Méthode selon l'une quelconque des revendications précédentes, dans laquelle ladite cellule *Bacillus* est sélectionnée dans le groupe consistant en *B. licheniformis, B. lentus, B. brevis, B. stearothermophilus, B. alkalophilus, B. amyloliquefaciens, B. coagulans, B. circulans, B. lautus, B. methanolicus, B. anthracis et B. thuringiensis.*

6. Méthode selon l'une quelconque des revendications 1 à 4, dans laquelle ladite cellule de Bacillus est *B. subtilis*.

7. Méthode selon l'une quelconque des revendications précédentes, dans laquelle ledit polypeptide est sélectionné dans le groupe consistant en hormone, enzyme, facteur de croissance et cytokine.

8. Méthode selon l'une quelconque des revendications précédentes, dans laquelle ledit polypeptide est hétérologue.

9. Méthode selon l'une quelconque des revendications précédentes, dans laquelle ledit polypeptide est une enzyme sélectionnée dans le groupe consistant en protéases, carbohydrates, réductases, lipases, isomérases, transférases, kinases phosphatases, cellulase, endoglucosidase H, oxydase, alpha-amylase, lignocellulose hémicellulase, pectinase et liginase.

10. Méthode selon l'une quelconque des revendications précédentes, dans laquelle ledit polypeptide est une bacillus protéase, une subtilisine, une amylase ou une bacillus amylase.

11. Méthode de production d'un polypeptide dans une cellule *Bacillus* comprenant les étapes de:
(a) obtenir une cellule Bacillus comprenant l'acide nucléique codant pour un polypeptide à produire, ladite cellule comprenant en outre un opéron de transport de dciA dipeptide, où au moins un produit de gène dudit opéron est inactif dans ladite cellule; et
(b) cultiver ladite cellule sous des conditions qui conviennent pour l'expression de sorte que ledit polypeptide est produit.

12. Méthode selon la revendication 11, dans laquelle ledit produit de gène est inactif par suite de la mutation dans ledit opéron.

13. Méthode selon la revendication 12, dans laquelle ladite mutation est une mutation de changement de phase ou est une délétion d'un ou de plusieurs nucléotides.

14. Méthode selon l'une quelconque des revendications 11 à 13, dans laquelle ledit produit de gène inactif est dciAE ou dciAA.

15. Méthode selon l'une quelconque des revendications 11 à 14, dans laquelle ledit polypeptide est hétérologue.

16. Méthode de production d'une cellule *Bacillus* ayant une sécrétion de polypeptide augmentée, comprenant l'inactivation d'un opéron de transport de dciA dipeptide dans ladite cellule.

17. Méthode selon la revendication 16, dans laquelle ledit opéron a été muté pour inactiver un produit de gène d'un gène dciAE ou d'un gène dciAA.
